# EUROPEAN PATENT APPLICATION

(11) **EP 3 910 254 A1**
(43) Date of publication of application: **17.11.2021**
(21) Application number: 20173804.4
(22) Date of filing: 11.05.2020
(51) Int. Cl.: F24F 6/12, F24F 3/16, A61L 9/14, C25B 1/13

(54) **AIR DISINFECTOR AND HUMIDIFIER COMBO**

(71) Applicant: BGT Materials Limited, Manchester M13 9PL (GB)
(72) Inventor: CHANG, Kuo-Hsin, CHIAYI COUNTY, 622 (TW); LAI, Chung-Ping, HSINCHU COUNTY 302 (TW)
(74) Representative: Cabinet Chaillot

(57) **Abstract**

An air disinfector and humidifier combo is revealed. The air disinfector and humidifier combo includes a tank used for storing water and provided with an air outlet, an electrolytic oxygen generator arranged at the tank for hydrolysis of water in the tank to generate ozone water, an ultrasonic oscillator disposed on the tank for atomizing the ozone water in the tank to produce ozone water mist, an air pump, a bubble refiner connected to an air vent of the air pump and mounted in the tank for generating submicron bubbles, and an electronic control unit electrically connected to the electrolytic oxygen generator, the ultrasonic oscillator, and the air pump for control of their operating time. The air disinfector and humidifier combo provides humidification, disinfection and purification functions. The submicron fine bubbles of the ozone water interact better with dirty air to improve disinfection and cleaning effect.

## Description

### BACKGROUND OF THE INVENTION

### Technical Field

The present invention relates to an air purifier, especially to an air disinfector and humidifier combo with humidification, disinfection and air purification functions.

### Description of Related Art

Ozone is a strong oxidant and disinfectant with good ability in disinfection and degradation of organic contaminants and disinfection effect of ozone on wastewater is enhanced by being used in combination with ultrasonic waves, as revealed in the research by HE Shi-Chuan, ZHU Chang-Ping, SHAN Ming-Lei, & FENG Ruo (2005.09), "Recent advances in wastewater treatment with ultrasonic-ozone method", Journal of Technical Acoustics, Vol. 24, No. 3 pp. 173-177.

Ozone can be produced via UV (ultraviolet) light, corona discharge (electrical discharge), or electrolysis. The most common ways are corona discharge (electrical discharge) and electrolytic ozone production. The corona discharge method produces gas mixture containing ozone (not pure ozone) and toxic nitrogen oxides as a by-product. There are two types of corona discharge ozone generators: the parallel-plate type and the shell and tube type. The parallel-plate type is used in small-capacity generator while the shell and tube type is employed in high-capacity commercial ozone generators. Recently electrolytic ozone production is widely used in ozone generators with hydrogen gas evolved in the cathode and oxygen gas evolved in the anode. The electrolytic ozone generator creates ozone with high concentration and no poisonous nitrogen oxides byproducts. Thus the electrolytic ozone generation technology has wide and promising applications.

The conventional sterilization and disinfection technology that combines ozone and ultrasonic waves has been used. Air is passed through an ozone generator to create ozone gas and then ozone gas is introduced into water to get ozone water. Next the ozone water is treated by ultrasonic atomization or spray head atomization for being applied to disinfection. Refer to Chinese Pat. Pub. No. CN1317344A, "disinfection method combining ultrasonic waves with ozone and device of the same", Chinese Utility Patent with the number of announcement of grant of patent right CN201006030 "ultrasonic atomized ozone disinfector" and Chinese Utility Patent with the number of announcement of grant of patent right CN2646601 "ultrasonic humidifier with disinfection function", all reveal similar techniques.

Moreover, refer to Chinese Utility Patent with the number of announcement of grant of patent right CN203413756U "disinfector and humidifier combo", a device in which ozone water is generated by hydrolysis and used for humidification and disinfection is revealed. The device includes a tank, an electrolytic cell, an atomizer (non-ultrasonic), a pneumatic member, a control circuit, etc. The tank and the electrolytic cell are communicating by a pipeline so that water is cycled between the tank and the electrolytic cell. Ozone water generated by hydrolysis in the electrolytic cell is turned back to the tank. A water outlet of the tank is connected to the atomizer for turning the ozone water into mist which is exhausted to the air by the pneumatic member. The electrolytic cell is a divided cell provided with an anode compartment and a cathode compartment separated from each other. The volume ratio of the anode compartment to the cathode compartment can be changed. The water in the cathode compartment is set to be collected in another tank. After being used for a period of time, the tank with the water collected needs to be drained and cleaned. Thus there is room for improvement and there is a need to provide an air disinfector and humidifier combo with novel structure.

### BRIEF DESCRIPTION OF THE DRAWINGS

Therefore it is a primary object of the present invention to provide an air disinfector and humidifier combo which provides humidification, disinfection and air purification functions.

In order to solve the problems mentioned above, an air disinfector and humidifier combo according to the present invention includes a tank, an electrolytic oxygen generator, an ultrasonic oscillator, an air pump, a bubble refiner and an electronic control unit. The tank is used for storing water and provided with an air outlet. The electrolytic oxygen generator is mounted in the tank for hydrolysis of water in the tank to generate ozone water. The ultrasonic oscillator is arranged at the tank for atomizing the ozone water in the tank into small droplets to get ozone water mist. An air vent of the air pump is connected to the bubble refiner which is disposed in the tank for producing fine bubbles in the tank. The electronic control unit is connected to the electrolytic oxygen generator, the ultrasonic oscillator, and the air pump for driving the electrolytic oxygen generator, the ultrasonic oscillator, and the air pump to work and also controlling their operating time.

Preferably, the tank consists of a main body with an opening and a detachable cover disposed on the opening. At least one air outlet is arranged at the cover.

Preferably, the bubble refiner produces the fine bubbles in submicron scale.

Preferably, an air inlet of the air pump is provided with a dust filter while a check valve is arranged between the air vent of the air pump and the bubble refiner.

Preferably, the ultrasonic oscillator is disposed on an outer side of the tank and the outer side of the tank includes an outer surface of a lateral side of the tank and an outer surface of a bottom side of the tank.

Preferably, the ultrasonic oscillator is disposed on an inner side of the tank and the inner side of the tank includes an inner surface of a lateral side of the tank and an inner surface of a bottom side of the tank.

Preferably, the electrolytic oxygen generator includes an anode plate and a cathode plate, both electrically connected to the electronic control unit. The electronic control unit controls operating time of the electrolytic oxygen generator to produce nano-scale ozone by hydrolysis of water at the anode plate and further generate the ozone water.

Preferably, the anode plate is made of active electrode materials with anti-oxidative activity selected from the group consisting platinum, stainless steel, titanium, insoluble anode materials (dimensionally stable anode (DSA)), graphite, graphene, doped and undoped oxides, nitrogen-doped or boron-doped diamond, and a combination thereof. The cathode plate is made of a material selected from the group consisting of platinum, stainless steel, titanium, graphite, graphene, stainless steel with a coating that promotes hydrogen generation, titanium with a coating that promotes hydrogen generation, and a combination thereof.

Preferably, the anode plate incudes a stainless steel electrode coated with the active electrode materials with the anti-oxidative activity and a titanium electrode coated with the active electrode materials with the anti-oxidative activity.

Preferably, an air intake is arranged at the bottom side of the tank and is connected to an outlet end of the check valve while the bubble refiner is connected to the outlet end of the check valve by the air intake. An inlet end of the check valve is connected to the air vent of the air pump by a tube connector. The inlet end of the check valve can be either connected to or separated from the tube connector.

Preferably, the air disinfector and humidifier combo further includes a base used for loading the tank and the tube connector is mounted on the base. The electrolytic oxygen generator and the ultrasonic oscillator both disposed on the tank are electrically connected to a first electrical connector while the electronic control unit is electrically connected to a second electrical connector. The first electrical connector is disposed on the outer side of the tank and the second electrical connector is arranged at the base while the first electrical connector and the second electrical connector are able to be plugged in and coupled to each other to form a circuit. The inlet end of the check valve on the bottom of the tank is directly connected to the tube connector when the tank is disposed on the base while the first electrical connector and the second electrical connector are plugged in and coupled to each other.

The present air disinfector and humidifier combo features on that the air disinfector and humidifier combo provides multiple functions including humidification, disinfection and air purification. The bubble refiner creates submicron fine bubbles which make ozone water interact better with dirty air to improve disinfection and cleaning effect of the ozone water on the dirty air. Moreover, the collision and mixture of the submicron bubbles with ozone nanobubbles are further enhanced by the ultrasonic oscillator. The disinfection is achieved by means of strong oxidizing power of the ozone.

The embodiments, advantages and functions of the present invention are described as follows with reference to the figures.

### BRIEF DESCRIPTION OF THE DRAWINGS

The structure and the technical means adopted by the present invention to achieve the above and other objects can be best understood by referring to the following detailed description of the preferred embodiments and the accompanying drawings, wherein:
Fig. 1 is a schematic drawing showing a sectional view of an embodiment according to the present invention;

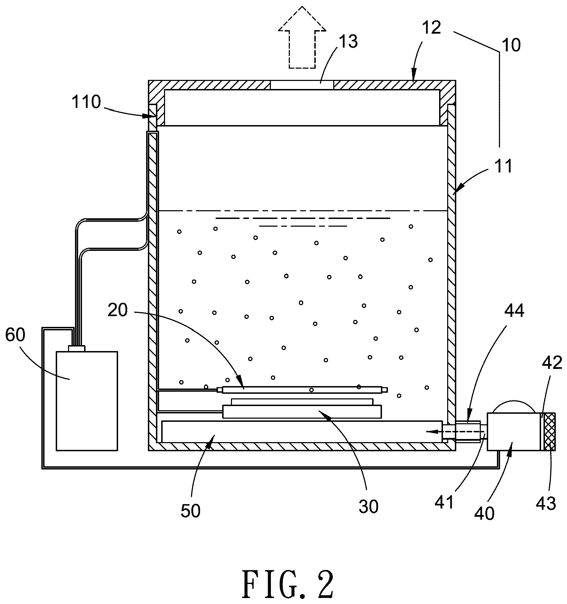

Fig. 2 is a schematic drawing showing a sectional view of another embodiment according to the present invention;
Fig. 3 is a schematic drawing showing a sectional view of a further embodiment in which a tank and a base are separated from each other according to the present invention;
Fig. 4 is a schematic drawing showing a sectional view of the embodiment in

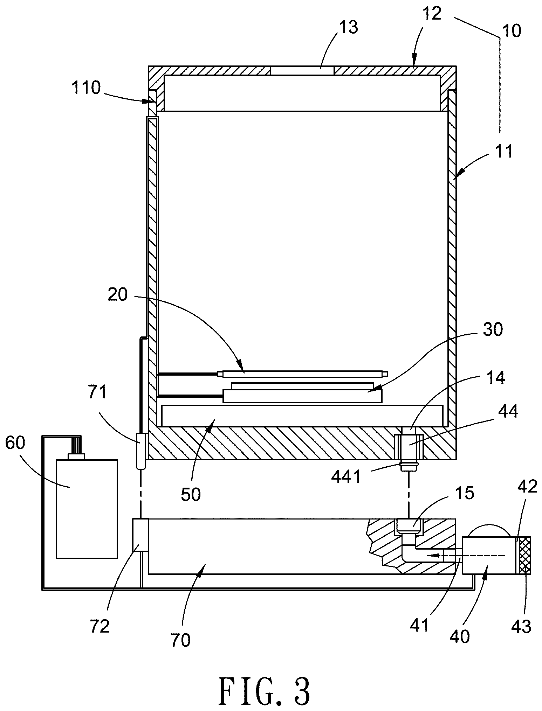

Fig. 3 in which the tank is mounted on the base and connected to an air pump by a check valve and a tube connector according to the present invention;

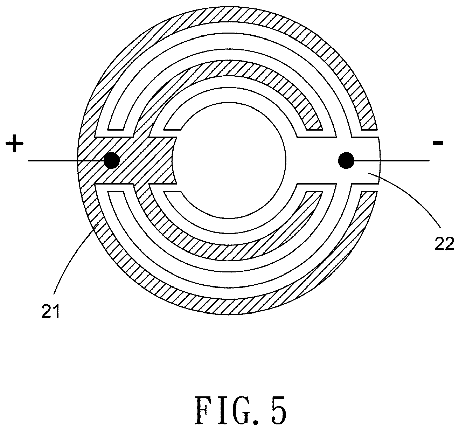

Fig. 5 is a schematic drawing showing an anode plate and a cathode plate in an electrolytic oxygen generator of an embodiment according to the present invention.

### DETAILED DESCRIPTION OF THE PREFERRED EMBODIMENT

The relative positions descried in the following embodiments, including upper, lower, left and right, unless otherwise specified, are based on the directions indicated by the components in the figures.

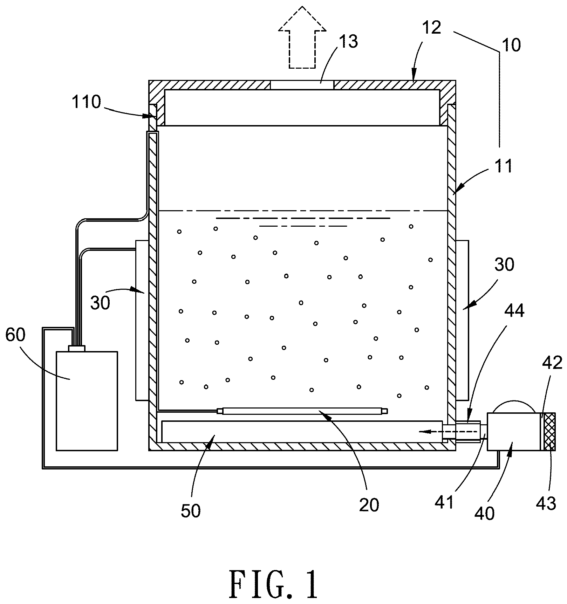

Refer to Fig. 1, a schematic drawing showing sectional view of an embodiment of an air disinfector and humidifier combo is revealed. The air disinfector and humidifier combo according to the present invention basically includes a tank 10, an electrolytic oxygen generator 20, an ultrasonic oscillator 30, an air pump 40, a bubble refiner 50 and an electronic control unit 60.

The tank 10 is used for storing water or aqueous solution containing electrolytes. In a preferred embodiment, the tank 10 includes a main body 11, a detachable cover 12 and at least one air outlet 13. The main body 11 has an opening 110 while the detachable cover 12 is disposed on the opening 110 and the air outlet 13 is arranged at the cover 12.

The electrolytic oxygen generator 20 is arranged at the main body 11 of the tank 10 for hydrolysis of the water in the tank 10 to generate ozone water. The electrolytic oxygen generator 20 basically includes an anode plate 21 and a cathode plate 22, both electrically connected to the electronic control unit 60, and a direct voltage or current is applied to two ends of both the anode plate 21 and the cathode plate 22. The electronic control unit 60 is used to drive the electrolytic oxygen generator 20 to work and control operating time of the electrolytic oxygen generator 20. Nano-scale ozone is produced on the anode plate 21 by hydrolysis and ozone water is further generated. There is no limit on the shape of the anode plate 21 and the cathode plate 22. The anode plate 21 and the cathode plate 22 shown in Fig. 5 are only an exemplary configuration. The structure of the anode plate 21 and the cathode plate 22 is not limited, as long as they are in close positions, their shapes are symmetrical, and the electric field generated by them is uniform. The shape of the anode plate 21 and the cathode plate 22 can be a column, a mesh, a sheet or other symmetrical shape.

The anode plate 21 is preferably to be made of active electrode materials with anti-oxidative activity including platinum, stainless steel, titanium, insoluble anode materials (such as dimensionally stable anode (DSA)), graphite/or graphene, doped and undoped oxides (such as RuO2, IrO2, PbO2, SnO2, TiO2, etc.), nitrogen-doped or boron-doped diamond, or their combinations. The main anode materials used in electrolysis that generates ozone water are PbO2, SnO2, and B-doped (boron-doped) diamond. Compared with platinum, the three materials all have high oxygen evolution overpotential (OEP) so that they can inhibit the oxygen evolution reaction, increase the voltage and improve the ozone generation efficiency. In a preferred embodiment, the anode plate 21 includes a stainless steel electrode/or a titanium electrode coated with the active electrode materials with anti-oxidative activity mentioned above. As to the cathode plate 22, it is preferably made of platinum, stainless steel, titanium, graphite/or graphene, stainless steel with a coating that promotes hydrogen generation (such as platinum), titanium with a coating that promotes hydrogen generation (such as platinum), or their combinations.

The ultrasonic oscillator 30 is arranged at the tank 10 for atomizing the ozone water in the tank 10 into small droplets to get ozone water mist with functions of humidification and disinfection. In a preferred embodiment, the ultrasonic oscillator 30 is disposed on the outer side of the tank 10 and the outer side includes the outer surface of the lateral side and the outer surface of the bottom side of the tank 10. Refer to Fig. 1, the ultrasonic oscillator 30 is disposed on the outer surface of the lateral side of the tank 10. By the ultrasonic oscillator 30, ultrasonic vibrations are applied to the ozone water in the tank 10 to produce ozone-water mist blown out of the water surface. In another preferred embodiment, the ultrasonic oscillator 30 is arranged at the inner side of the tank 10 and the inner side includes the inner surface of the lateral sides and the inner surface of the bottom side. In the embodiment shown in Fig, 2, the ultrasonic vibration 30 is installed on the inner surface of the bottom side of the tank 10.

An air vent 41 of the air pump 40 is connected to the bubble refiner 50 which is disposed in the tank 10 for producing fine bubbles. In a preferred embodiment, a check valve 44 is arranged between the air vent 41 of the air pump 40 and the bubble refiner 50 for preventing the water in the tank 10 from returning to the air pump 40. The check valve 44 is not required once the height of the air pump 40 is above the highest water level in the tank 10. An air inlet 42 of the air pump 40 is preferably provided with a dust filter 43 which is used for filtering larger particles in the air. Thus the amount of pollutants, dust and particulates flowing from the air to the tank 10 is reduced. In a preferred embodiment, the bubble refiner 50 generates submicron bubbles. Thus the bubbles of the ozone water interact better with dirty air to improve disinfection and cleaning effect of the ozone water on the dirty air. Moreover, the collision and mixture of the submicron bubbles with ozone nanobubbles are further enhanced by the ultrasonic oscillator 30. The ozone water mist generated is exhausted to the environment through the air outlet 13. Thereby the disinfection function is provided by means of strong oxidizing power of the ozone.

The electronic control unit 60 is electrically connected to the electrolytic oxygen generator 20, the ultrasonic oscillator 30, and the air pump 40 for driving the electrolytic oxygen generator 20, the ultrasonic oscillator 30, and the air pump 40 to work. Moreover, the electronic control unit 60 can also control the operating time of the electrolytic oxygen generator 20, the ultrasonic oscillator 30, and the air pump 40 separately.

In a preferred embodiment, the tank 10 is designed in a detachable manner and users can easily take off the tank 10 for cleaning. As shown in Fig. 3, an air intake 14 is located on the bottom side of the tank 10 and is connected to an outlet end of the check valve 44 while the bubble refiner 50 is connected to the outlet end of the check valve 44 by the air intake 14. An inlet end of the check valve 44 is connected to the air vent 41 of the air pump 40 through a tube connector 15. The inlet end of the check valve 44 can be either connected to or separated from the tube connector 15. For example, a plug-in design is adopted. A leak-proof rubber ring 441 is mounted on outer surface of the inlet end of the check valve 44 and the inlet end of the check valve 44 can be directly plugged into the tube connector 15 for connection and the connection completed is leak-proof.

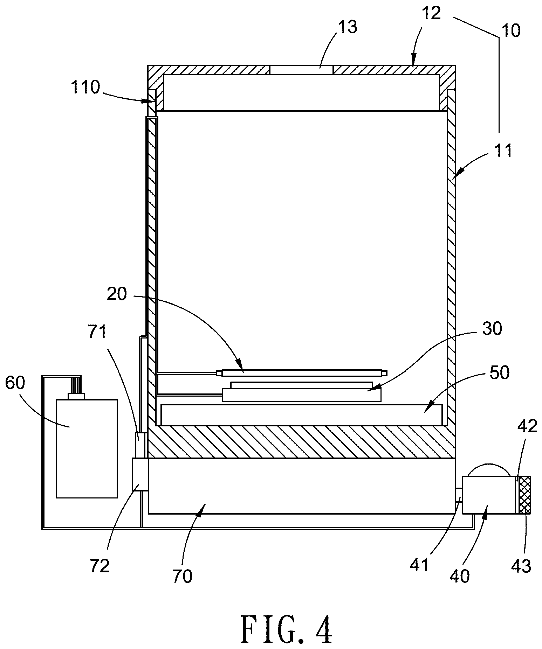

Refer to Fig. 3, a further embodiment is revealed. In this embodiment, the air disinfector and humidifier combo further includes a base 70 used for loading the tank 10 and the tube connector 15 is mounted on the base 70. The electrolytic oxygen generator 20 and the ultrasonic oscillator 30 both disposed on the tank 10 are electrically connected to a first electrical connector 71 while the electronic control unit 60 is electrically connected to a second electrical connector 72. The first electrical connector 71 is disposed on the outer side of the tank 10 and the second electrical connector 72 is arranged at the base 70. The first electrical connector 71 and the second electrical connector 72 can be plugged in and coupled to each other to form a circuit. Also refer to Fig. 4, the inlet end of the check valve 44 on the bottom of the tank 10 is directly connected to the tube connector 15 when the tank 10 is disposed on the base 70. At the same time, the first electrical connector 71 and the second electrical connector 72 are plugged in and coupled to each other. On the other hand, the tank 10 is removed from the base 70 and the inlet end of the check valve 44 is separated from the tube connector 15 on the base 70 once users need to take off the tank 10 for cleaning. The first electrical connector 71 and the second electrical connector 72 are also separated. Thereby the assembly and disassembly of the tank 10 are easy and convenient. After the long-term use, the amount of the water in the tank 10 is reduced due to the function of humidification. Thus there is a need to fill water into the tank 10. Besides easy cleaning, the detachable design of the tank 10 also solves the problems of residual contaminants or impurities in the tank 10. The ultrasonic oscillator 30 is preferably arranged at the outer surface of the lateral side and the outer surface of the bottom side of a wall of the tank 10.

Additional advantages and modifications will readily occur to those skilled in the art. Therefore, the invention in its broader aspects is not limited to the specific details, and representative devices shown and described herein. Accordingly, various modifications may be made without departing from the spirit or scope of the general inventive concept as defined by the appended claims and their equivalent.

## Claims

1. An air disinfector and humidifier combo comprising:
a tank (10) used for storing water and provided with at least one air outlet (13);
an electrolytic oxygen generator (20) which is mounted in the tank (10) for hydrolysis of water in the tank (10) to generate ozone water;
an ultrasonic oscillator (30) which is arranged at the tank (10) for atomizing the ozone water in the tank (10) into small droplets to get ozone water mist;
an air pump (40) having an air vent (41) connected to a bubble refiner (50);
the bubble refiner (50) disposed in the tank (10) for producing fine bubbles in the tank (10); and
an electronic control unit (60) which is connected to the electrolytic oxygen generator (20), the ultrasonic oscillator (30), and the air pump (40) for driving the electrolytic oxygen generator (20), the ultrasonic oscillator (30), and the air pump (40) to work and controlling operating time of the electrolytic oxygen generator (20), the ultrasonic oscillator (30), and the air pump (40).

2. The device as claimed in claim 1, wherein the tank (10) includes a main body (11) with an opening (110) and a detachable cover (12) disposed on the opening (110); the air outlet (13) is arranged at the cover (12).

3. The device as claim in claim 1, wherein the bubble refiner (50) produces the fine bubbles in submicron scale.

4. The device as claim in claim 1, wherein an air inlet (42) of the air pump (40) is provided with a dust filter (43) while a check valve (44) is arranged between the air vent (41) of the air pump (40) and the bubble refiner (50).

5. The device as claim in claim 1, wherein the ultrasonic oscillator (30) is disposed on an outer side of the tank (10) and the outer side of the tank (10) includes an outer surface of a lateral side of the tank (10) and an outer surface of a bottom side of the tank (10).

6. The device as claim in claim 1, wherein the ultrasonic oscillator (30) is disposed on an inner side of the tank (10) while the inner side of the tank (10) includes an inner surface of a lateral side of the tank (10) and an inner surface of a bottom side of the tank (10).

7. The device as claim in claim 1, wherein the electrolytic oxygen generator (20) includes an anode plate (21) and a cathode plate (22), both electrically connected to the electronic control unit (60); the electronic control unit (60) is used to control operating time of the electrolytic oxygen generator (20) to produce nano-scale ozone by hydrolysis of water at the anode plate (21) and further generate the ozone water.

8. The device as claim in claim 7, wherein the anode plate (21) is made of active electrode materials with anti-oxidative activity which is selected from the group consisting platinum, stainless steel, titanium, insoluble anode materials (dimensionally stable anode (DSA)), graphite, graphene, doped and undoped oxides, nitrogen-doped diamond, boron-doped diamond and a combination thereof; the cathode plate (22) is made of a material selected from the group consisting of platinum, stainless steel, titanium, graphite, graphene, stainless steel with a coating that promotes hydrogen generation, titanium with a coating that promotes hydrogen generation, and a combination thereof.

9. The device as claim in claim 8, wherein the anode plate (21) includes a stainless steel electrode coated with the active electrode materials with the anti-oxidative activity on surface and a titanium electrode coated with the active electrode materials with the anti-oxidative activity on surface.

10. The device as claim in claim 1, wherein the an air intake (14) is located on the bottom side of the tank (10) and is connected to an outlet end of the check valve (44) while the bubble refiner (50) is connected to the outlet end of the check valve (44) by the air intake (14); an inlet end of the check valve (44) is connected to the air vent (41) of the air pump (40) by a tube connector (15); the inlet end of the check valve (44) is able to be connected to or separated from the tube connector (15).

11. The device as claim in claim 10, wherein the air disinfector and humidifier combo further includes a base (70) used for loading the tank (10) and the tube connector (15) is mounted on the base (70); the electrolytic oxygen generator (20) and the ultrasonic oscillator (30) both disposed on the tank (10) are electrically connected to a first electrical connector (71) while the electronic control unit (60) is electrically connected to a second electrical connector (72); the first electrical connector (71) is disposed on the outer side of the tank (10) and the second electrical connector (72) is arranged at the base (70) while the first electrical connector (71) and the second electrical connector (72) are able to be plugged in and coupled to each other to form a circuit; the inlet end of the check valve (44) on the bottom of the tank (10) is directly connected to the tube connector (15) when the tank (10) is disposed on the base (70) while the first electrical connector (71) and the second electrical connector (72) are plugged in and coupled to each other.
